# EUROPEAN PATENT SPECIFICATION

(11) **EP 2 931 138 B1**
(45) Date of publication and mention of the grant of the patent: **15.05.2019**
(21) Application number: 13808415.7
(22) Date of filing: 04.12.2013
(51) Int. Cl.: A61B 17/064, A61B 17/115

(54) **SURGICAL STAPLER WITH VARYING STAPLE WIDTHS ALONG DIFFERENT CIRCUMFERENCES**
CHIRURGISCHE KLAMMERVORRICHTUNG MIT VARIIERENDER KLAMMERBREITE ENTLANG UNTERSCHIEDLICHER UMFÄNGE
AGRAFEUSE CHIRURGICALE AYANT DES LARGEURS D'AGRAFE VARIABLES LE LONG DE DIFFÉRENTES CIRCONFÉRENCES

(30) Priority: 06.12.2012 US 201213706827
(43) Date of publication of application: 21.10.2015
(73) Proprietor: Ethicon LLC, 00969 Guaynabo (PR)
(72) Inventor: MEASAMER, John, P., Cincinnati, OH 45249 (US); ALEXANDER, Johnny, H., III, West Chester, OH 45069 (US); MILLER, Christopher, C., Loveland, OH 45140 (US); LESKO, Jason, R., Harrison, OH 45030 (US); MERRITT, James, S., El Paso, TX 79912 (US); WIDENHOUSE, Tamara S., Vetro, Clarksville, OH 45113 (US); SHELTON, Frederick, E., IV., Hillsboro, OH 45133 (US)
(74) Representative: Carpmaels & Ransford LLP
(86) International application number: PCT/US2013/073100
(87) International publication number: WO 2014/089200

(56) References cited:
- EP-A2- 2 436 318
- US-A1- 2007 034 666
- US-A1- 2009 005 809
- US-A1- 2012 080 498
- US-A1- 2012 193 398

## Description

### BACKGROUND

In some settings, a surgeon may want to position a surgical instrument through an orifice of the patient and use the instrument to adjust, position, attach, and/or otherwise interact with tissue within the patient. For instance, in some surgical procedures, portions of the gastrointestinal tract may be cut and removed to eliminate undesirable tissue or for other reasons. Once the desired tissue is removed, the remaining portions may need to be recoupled together. One such tool for accomplishing these anastomotic procedures is a circular stapler that is inserted through a patient's orifice.

Examples of circular surgical staplers are described in U.S. Pat. No. 5,205,459, entitled "Surgical Anastomosis Stapling Instrument," issued April 27, 1993; U.S. Pat. No. 5,271,544, entitled "Surgical Anastomosis Stapling Instrument," issued December 21, 1993; U.S. Pat. No. 5,275,322, entitled "Surgical Anastomosis Stapling Instrument," issued January 4, 1994; U.S. Pat. No. 5,285,945, entitled "Surgical Anastomosis Stapling Instrument," issued February 15, 1994; U.S. Pat. No. 5,292,053, entitled "Surgical Anastomosis Stapling Instrument," issued March 8, 1994; U.S. Pat. No. 5,333,773, entitled "Surgical Anastomosis Stapling Instrument," issued August 2, 1994; U.S. Pat. No. 5,350,104, entitled "Surgical Anastomosis Stapling Instrument," issued September 27, 1994; and U.S. Pat. No. 5,533,661, entitled "Surgical Anastomosis Stapling Instrument," issued July 9, 1996. Some such staplers are operable to clamp down on layers of tissue, cut through the clamped layers of tissue, and drive staples through the layers of tissue to substantially seal the severed layers of tissue together near the severed ends of the tissue layers, thereby joining two severed ends of an anatomical lumen.

Merely additional other exemplary surgical staplers are disclosed in U.S. Pat. No. 4,805,823, entitled "Pocket Configuration for Internal Organ Staplers," issued February 21, 1989; U.S. Pat. No. 5,415,334, entitled "Surgical Stapler and Staple Cartridge," issued May 16, 1995; U.S. Pat. No. 5,465,895, entitled "Surgical Stapler Instrument," issued November 14, 1995; U.S. Pat. No. 5,597,107, entitled "Surgical Stapler Instrument," issued January 28, 1997; U.S. Pat. No. 5,632,432, entitled "Surgical Instrument," issued May 27, 1997; U.S. Pat. No. 5,673,840, entitled "Surgical Instrument," issued October 7, 1997; U.S. Pat. No. 5,704,534, entitled "Articulation Assembly for Surgical Instruments," issued January 6, 1998; U.S. Pat. No. 5,814,055, entitled "Surgical Clamping Mechanism," issued September 29, 1998; U.S. Pat. No. 6,978,921, entitled "Surgical Stapling Instrument Incorporating an E-Beam Firing Mechanism," issued December 27, 2005; U.S. Pat. No. 7,000,818, entitled "Surgical Stapling Instrument Having Separate Distinct Closing and Firing Systems," issued February 21, 2006; U.S. Pat. No. 7,143,923, entitled "Surgical Stapling Instrument Having a Firing Lockout for an Unclosed Anvil," issued December 5, 2006; U.S. Pat. No. 7,303,108, entitled "Surgical Stapling Instrument Incorporating a Multi-Stroke Firing Mechanism with a Flexible Rack," issued December 4, 2007; U.S. Pat. No. 7,367,485, entitled "Surgical Stapling Instrument Incorporating a Multistroke Firing Mechanism Having a Rotary Transmission," issued May 6, 2008; U.S. Pat. No. 7,380,695, entitled "Surgical Stapling Instrument Having a Single Lockout Mechanism for Prevention of Firing," issued June 3, 2008; U.S. Pat. No. 7,380,696, entitled "Articulating Surgical Stapling Instrument Incorporating a Two-Piece E-Beam Firing Mechanism," issued June 3, 2008; U.S. Pat. No. 7,404,508, entitled "Surgical Stapling and Cutting Device," issued July 29, 2008; U.S. Pat. No. 7,434,715, entitled "Surgical Stapling Instrument Having Multistroke Firing with Opening Lockout," issued October 14, 2008; and U.S. Pat. No. 7,721,930, entitled "Disposable Cartridge with Adhesive for Use with a Stapling Device," issued May 25, 2010. While the surgical staplers referred to above are described as being used in endoscopic procedures, it should be understood that such surgical staplers may also be used in open procedures and/or other non-endoscopic procedures. US 2012/0080498 A1 discloses an apparatus according to the preamble of claim 1.

While various kinds of surgical stapling instruments and associated components have been made and used, it is believed that no one prior to the inventor(s) has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a side elevation view of an exemplary circular stapling surgical instrument;
FIG. 2A depicts an enlarged longitudinal cross-section view of an exemplary stapling head assembly of the instrument of FIG. 1 showing an exemplary anvil in an open position;
FIG. 2B depicts an enlarged longitudinal cross-sectional view of the stapling head assembly of FIG. 2A showing the anvil in a closed position;
FIG. 2C depicts an enlarged longitudinal cross-sectional view of the stapling head assembly of FIG. 2A showing an exemplary staple driver and blade in a fired position;
FIG. 3 depicts an enlarged partial cross-sectional view of an exemplary staple formed against the anvil;
FIG. 4A depicts an enlarged side elevation view of an exemplary actuator handle assembly of the surgical instrument of FIG. 1 with a portion of the body removed, showing a trigger in an unfired position and a lockout feature in a locked position;
FIG. 4B depicts an enlarged side elevation view of the actuator handle assembly of FIG. 4A, showing the trigger in a fired position and the lockout feature in an unlocked position;
FIG. 5 depicts an enlarged partial perspective view of an exemplary indicator assembly of the surgical instrument of FIG. 1 showing an indicator window and indicator lever;
FIG. 6 depicts an diagrammatic view of the indicator window of FIG. 5 showing an exemplary indicator bar and exemplary corresponding staple representations;
FIG. 7 depicts an enlarged perspective view of an exemplary staple cartridge for use with the stapling head assembly of FIG. 2A;
FIG. 8 depicts a side elevation view of an exemplary large staple for use with the staple cartridge of FIG. 7;
FIG. 9 depicts a side elevation view of an exemplary medium staple for use with the staple cartridge of FIG. 7;
FIG. 10 depicts a side elevation view of an exemplary small staple for use with the staple cartridge of FIG. 7;
FIG. 11 depicts an enlarged top view of the staple cartridge of FIG. 7 with large staples, medium staples, and small staples loaded into the staple cartridge;
FIG. 12 depicts an enlarged perspective view of an exemplary staple support element;
FIG. 13 depicts a side elevation view of an exemplary outer staple outfitted with the staple support element of FIG. 12;
FIG. 14 depicts a side elevation view of an exemplary middle staple outfitted with a medium staple support element;
FIG. 15 depicts a side elevation view of an exemplary inner staple outfitted with a small staple support element;
FIG. 16 depicts an enlarged top view of an exemplary staple cartridge loaded with large staples, medium staples, and small staples outfitted with staple support element;
FIG. 17 depicts an enlarged top view of an alternative exemplary staple support element with longitudinally extending tabs;
FIG. 18 depicts an enlarged top view of an alternative exemplary staple support element with transversely extending tabs;
FIG. 19 depicts an enlarged top view of an alternative exemplary staple support element with transversely and longitudinally extending tabs; and
FIG. 20 depicts a perspective view of the staple support element of FIG. 19 in an exemplary staple cartridge.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

### I. Overview of Exemplary Circular Stapling Surgical Instrument

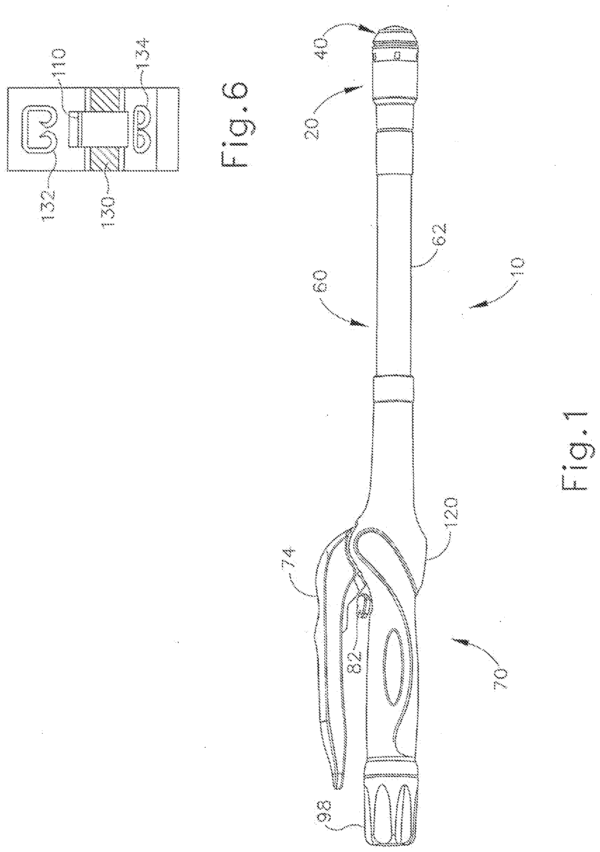

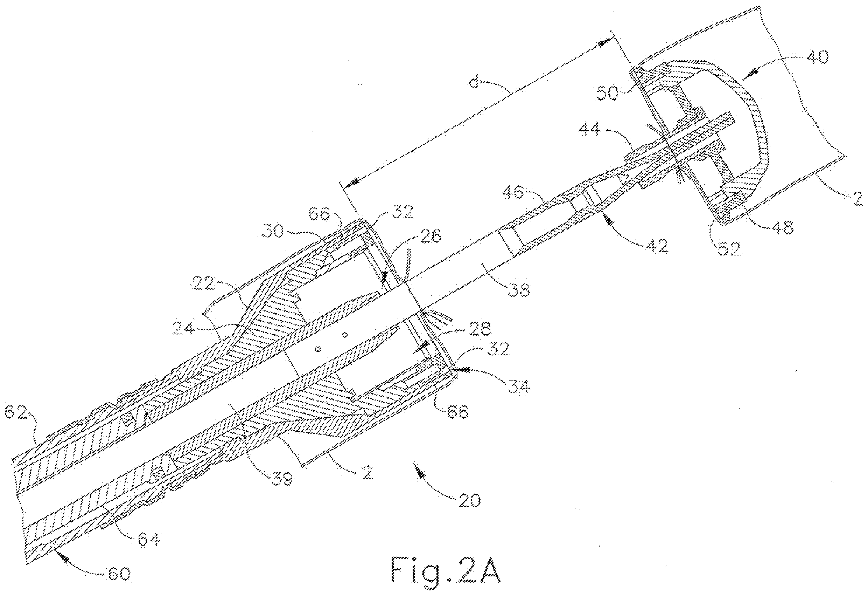

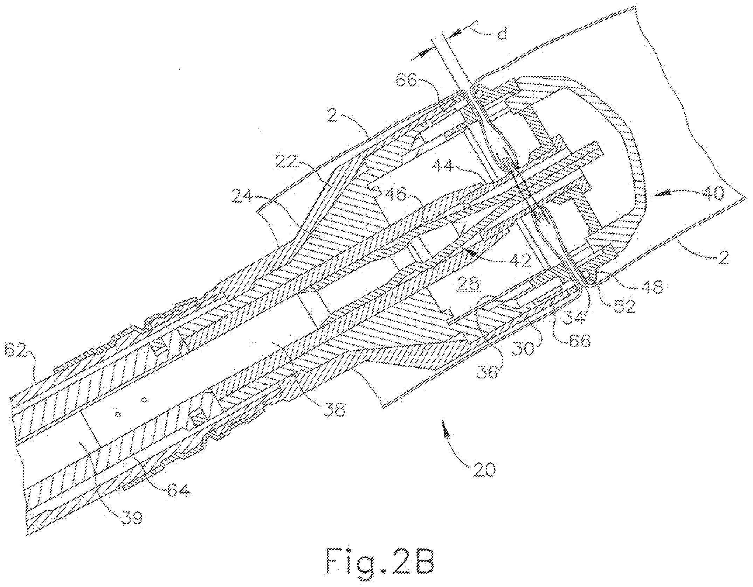

FIGS. 1-6 depict an exemplary circular surgical stapling instrument (10) having a stapling head assembly (20), a shaft assembly (60), and an actuator handle assembly (70), each of which will be described in more detail below. Shaft assembly (60) extends distally from actuator handle assembly (70) and stapling head assembly (20) is coupled to a distal end of shaft assembly (60). In brief, actuator handle assembly (70) is operable to actuate a staple driver (24) of stapling head assembly (20) to drive a plurality of staples (66) out of stapling head assembly (20). Staples (66) are bent to form completed staples by an anvil (40) that is attached at the distal end of instrument (10). Accordingly, tissue (2), shown in FIGS. 2A-2C, may be stapled utilizing instrument (10).

In the present example, instrument (10) comprises a closure system and a firing system. The closure system comprises a trocar (38), a trocar actuator (39), and a rotating knob (98). An anvil (40) may be coupled to a distal end of trocar (38). Rotating knob (98) is operable to longitudinally translate trocar (38) relative to stapling head assembly (20), thereby translating anvil (40) when anvil (40) is coupled to trocar (38), to clamp tissue between anvil (40) and stapling head assembly (20). The firing system comprises a trigger (74), a trigger actuation assembly (84), a driver actuator (64), and a staple driver (24). Staple driver (24) includes a knife (36) configured to sever tissue when staple driver (24) is actuated longitudinally. In addition, staples (66) are positioned distal to a plurality of staple driving members (30) of staple driver (24) such that staple driver (24) also drives staples (66) distally when staple driver (24) is actuated longitudinally. Thus, when trigger (74) is actuated and trigger actuation assembly (84) actuates staple driver (24) via driver actuator (64), knife (36) and members (30) substantially simultaneously sever tissue (2) and drive staples (66) distally relative to stapling head assembly (20) into tissue. The components and functionalities of the closure system and firing system will now be described in greater detail.

### A. Exemplary Anvil

As shown in FIGS. 1-2C, anvil (40) is selectively coupleable to instrument (10) to provide a surface against which staples (66) may be bent to staple material contained between stapling head assembly (20) and anvil (40). Anvil (40) of the present example is selectively coupleable to a trocar or pointed rod (38) that extends distally relative to stapling head assembly (20). Referring to FIGS. 2A-2C, anvil (40) is selectively coupleable via the coupling of a proximal shaft (42) of anvil (40) to a distal tip of trocar (38). Anvil (40) comprises a generally circular anvil head (48) and a proximal shaft (42) extending proximally from anvil head (48). In the example shown, proximal shaft (42) comprises a tubular member (44) having resiliently biased retaining clips (46) to selectively couple anvil (40) to trocar (38), though this is merely optional, and it should be understood that other retention features for coupling anvil (40) to trocar (38) may be used as well. For example, C-clips, clamps, threading, pins, adhesives, etc. may be employed to couple anvil (40) to trocar (38). In addition, while anvil (40) is described as selectively coupleable to trocar (38), in some versions proximal shaft (42) may include a one-way coupling feature such that anvil (40) cannot be removed from trocar (38) once anvil (40) is attached. Merely exemplary one-way features include barbs, one way snaps, collets, collars, tabs, bands, etc. Of course still other configurations for coupling anvil (40) to trocar (38) will be apparent to one of ordinary skill in the art in view of the teachings herein. For instance, trocar (38) may instead be a hollow shaft and proximal shaft (42) may comprise a sharpened rod that is insertable into the hollow shaft.

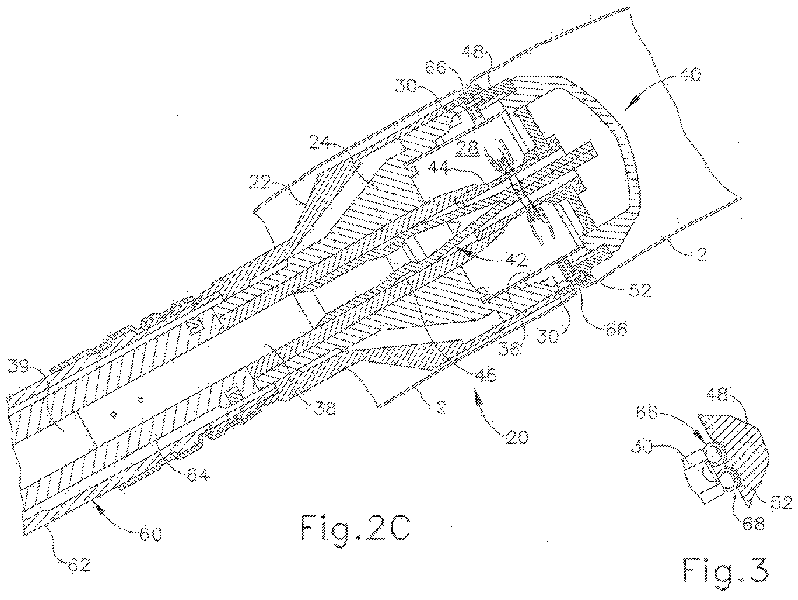

Anvil head (48) of the present example comprises a plurality of staple forming pockets (52) formed in a proximal face (50) of anvil head (48). Accordingly, when anvil (40) is in the closed position and staples (66) are driven out of stapling head assembly (20) into staple forming pockets (52), as shown in FIG. 2C, legs (68) of staples (66) are bent to form completed staples. It should be understood that staple forming pockets (52) are merely optional and may be omitted in some versions.

With anvil (40) as a separate component, it should be understood that anvil (40) may be inserted and secured to a portion of tissue (2) prior to being coupled to stapling head assembly (20). By way of example only, anvil (40) may be inserted into and secured to a first tubular portion of tissue (2) while instrument (10) is inserted into and secured to a second tubular portion of tissue (2). For instance, the first tubular portion of tissue (2) may be sutured to or about a portion of anvil (40), and the second tubular portion of tissue (2) may be sutured to or about trocar (38).

As shown in FIG. 2A, anvil (40) is then coupled to trocar (38). Trocar (38) of the present example is shown in a distal most actuated position. Such an extended position for trocar (38) may provide a larger area to which tissue (2) may be coupled prior to attachment of anvil (40). In addition, the extended position of trocar (38) may also provide for easier attachment of anvil (40) to trocar (38). Trocar (38) further includes a tapered distal tip. Such a tip may be capable of piercing through tissue and/or aiding the insertion of anvil (40) on to trocar (38), though the tapered distal tip is merely optional. For instance, in other versions trocar (38) may have a blunt tip. In addition, or in the alternative, trocar (38) may include a magnetic portion (not shown) which may attract anvil (40) towards trocar (38). Of course still further configurations and arrangements for anvil (40) and trocar (38) will be apparent to one of ordinary skill in the art in view of the teachings herein.

When anvil (40) is coupled to trocar (38), the distance between a proximal face of the anvil (40) and a distal face of stapling head assembly (20) defines a gap distance *d.* Trocar (38) of the present example is translatable longitudinally relative to stapling head assembly (20) via an adjusting knob (98) located at a proximal end of actuator handle assembly (70), as will be described in greater detail below. Accordingly, when anvil (40) is coupled to trocar (38), rotation of adjusting knob (98) enlarges or reduces gap distance *d* by actuating anvil (40) relative to stapling head assembly (20). For instance, as shown sequentially in FIGS. 2A-2B, anvil (40) is shown actuating proximally relative to actuator handle assembly (70) from an initial, open position to a closed position, thereby reducing the gap distance *d* and the distance between the two portions of tissue (2) to be joined. Once the gap distance *d* is brought within a predetermined range, stapling head assembly (20) may be fired, as shown in FIG. 2C, to staple and sever tissue (2) between anvil (40) and stapling head assembly (20). Stapling head assembly (20) is operable to staple and sever tissue (2) by a user pivoting a trigger (74) of actuator handle assembly (70), as will be described in greater detail below.

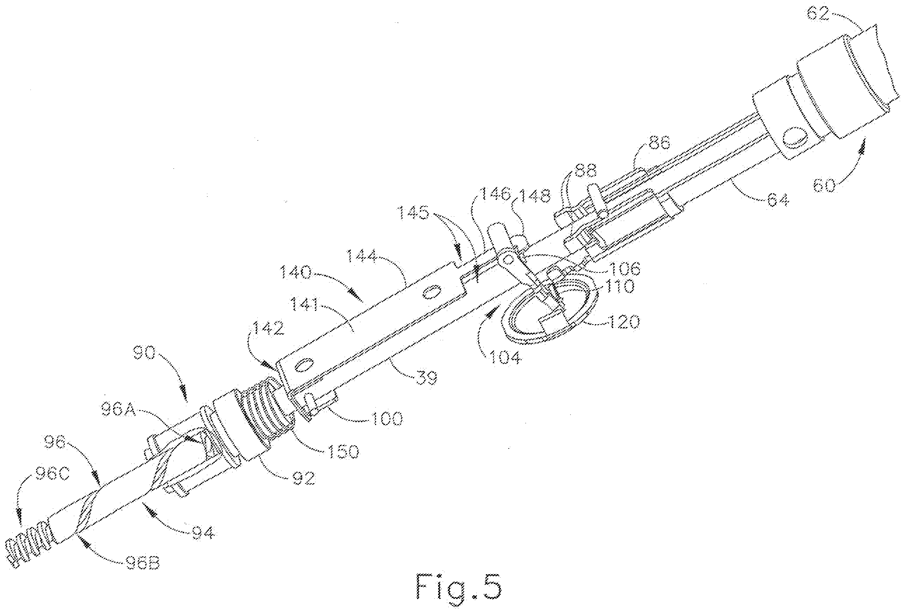

As noted above, gap distance *d* corresponds to the distance between anvil (40) and stapling head assembly (20). When instrument (10) is inserted into a patient, this gap distance *d* may not be easily viewable. Accordingly, a moveable indicator bar (110), shown in FIGS. 5-6, is provided to be visible through an indicator window (120) positioned opposite to trigger (74). Indicator bar (110) is operable to move in response to rotation of adjusting knob (98) such that the position of indicator bar (110) is representative of the gap distance *d.* As shown in FIG. 6, indicator window (120) further comprises a scale (130) which indicates that the anvil gap is within a desired operating range (e.g., a green colored region or "green zone") and a corresponding staple compression representation at each end of scale (130). By way of example only, as shown in FIG. 6, a first staple image (132) depicts a large staple height while a second staple image (134) depicts a small staple height. Accordingly, a user can view the position of the coupled anvil (40) relative to the stapling head assembly (20) via indicator bar (110) and scale (130). The user may then adjust the positioning of anvil (40) via adjusting knob (98) accordingly.

Referring back to FIGS. 2A-2C, a user sutures a portion of tissue (2) about tubular member (44) such that anvil head (48) is located within a portion of the tissue (2) to be stapled. When tissue (2) is attached to anvil (40), retaining clips (46) and a portion of tubular member (44) protrude out from tissue (2) such that the user may couple anvil (40) to trocar (38). With tissue (2) coupled to trocar (38) and/or another portion of stapling head assembly (20), the user attaches anvil (40) to trocar (38) and actuates anvil (40) proximally towards stapling head assembly (20) to reduce the gap distance *d.* Once instrument (10) is within the operating range, the user then staples together the ends of tissue (2), thereby forming a substantially contiguous tubular portion of tissue (2).

Anvil (40) may be further constructed in accordance with at least some of the teachings of U.S. Pat. No. 5,205,459; U.S. Pat. No. 5,271,544; U.S. Pat. No. 5,275,322; U.S. Pat. No. 5,285,945; U.S. Pat. No. 5,292,053; U.S. Pat. No. 5,333,773; U.S. Pat. No. 5,350,104; U.S. Pat. No. 5,533,661; and/or in accordance with other configurations as will be apparent to one of ordinary skill in the art in view of the teachings herein.

### B. Exemplary Stapling Head Assembly

Stapling head assembly (20) of the present example is coupled to a distal end of shaft assembly (60) and comprises a tubular casing (22) housing a slidable staple driver (24) and a plurality of staples (66) contained within staple pockets (32). Staples (66) and staple pockets (32) are disposed in a circular array about tubular casing (22). In the present example, staples (66) and staple pockets (32) are disposed in a pair of concentric annular rows of staples (66) and staple pockets (32). Staple driver (24) is operable to actuate longitudinally within tubular casing (22) in response to rotation of trigger (74) of actuator handle assembly (70). As shown in FIGS. 2A-2C, staple driver (24) comprises a flared cylindrical member having a trocar opening (26), a central recess (28), and a plurality of members (30) disposed circumferentially about central recess (28) and extending distally relative to shaft assembly (60). Each member (30) is configured to contact and engage a corresponding staple (66) of the plurality of staples (66) within staple pockets (32). Accordingly, when staple driver (24) is actuated distally relative to actuator handle assembly (70), each member (30) drives a corresponding staple (66) out of its staple pocket (32) through a staple aperture (34) formed in a distal end of tubular casing (22). Because each member (30) extends from staple driver (24), the plurality of staples (66) are driven out of stapling head assembly (20) at substantially the same time. When anvil (40) is in the closed position, staples (66) are driven into staple forming pockets (52) to bend legs (68) of the staples (66), thereby stapling the material located between anvil (40) and stapling head assembly (20). FIG. 3 depicts one merely exemplary staple (66) driven by a member (30) into a staple forming pocket (32) of anvil (40) to bend legs (68).

Staple driver (24) further includes a cylindrical knife (36) that is coaxial to trocar opening (26) and inset from staple pockets (32). In the present example, cylindrical knife (36) is disposed within central recess (28) to translate distally with staple driver (24). When anvil (40) is secured to trocar (38), as described above, anvil head (48) provides a surface against which cylindrical knife (36) cuts the material contained between anvil (40) and stapling head assembly (20). In some versions, anvil head (48) may include a recess (not shown) for cylindrical knife (36) to aid in cutting the material (e.g., by providing a cooperative shearing edge). In addition, or in the alternative, anvil head (48) may include one or more opposing cylindrical knives (not shown) offset from cylindrical knife (36) such that a scissor-type cutting action may be provided. Still other configurations will be apparent to one of ordinary skill in the art in view of the teachings herein. Stapling head assembly (20) is thus operable to both staple and cut tissue (2) substantially simultaneously in response to actuation by actuator handle assembly (70).

Of course stapling head assembly (20) may be further constructed in accordance with at least some of the teachings of U.S. Pat. No. 5,205,459; U.S. Pat. No. 5,271,544; U.S. Pat. No. 5,275,322; U.S. Pat. No. 5,285,945; U.S. Pat. No. 5,292,053; U.S. Pat. No. 5,333,773; U.S. Pat. No. 5,350,104; U.S. Pat. No. 5,533,661; and/or in accordance with other configurations as will be apparent to one of ordinary skill in the art in view of the teachings herein.

As noted previously, staple driver (24) includes a trocar opening (26). Trocar opening (26) is configured to permit trocar (38) to longitudinally slide relative to stapling head assembly (20) and/or shaft assembly (60). As shown in FIGS. 2A-2C, trocar (38) is coupled to a trocar actuator (39) such that trocar (38) can be actuated longitudinally via rotation of rotating knob (98), as will be described in greater detail below in reference to actuator handle assembly (70). In the present example, trocar actuator (39) comprises an elongated, relatively stiff shaft coupled to trocar (38), though this is merely optional. In some versions, actuator (39) may comprise a longitudinally stiff material while permitting lateral bending such that portions of instrument (10) may be selectively bent or curved during use; or instrument (10) may include a preset bent shaft assembly (60). One merely exemplary material is nitinol. When anvil (40) is coupled to trocar (38), trocar (38) and anvil (40) are translatable via actuator (39) to adjust the gap distance d between anvil (40) and stapling head assembly (20). Still further configurations for actuator (39) to longitudinally actuate trocar (38) will be apparent to one of ordinary skill in the art in view of the teachings herein.

### C. Exemplary Shaft Assembly

Stapling head assembly (20) and trocar (38) are positioned at a distal end of shaft assembly (60), as shown in FIGS. 2A-2C. Shaft assembly (60) of the present example comprises an outer tubular member (62) and a driver actuator (64). Outer tubular member (62) is coupled to tubular casing (22) of stapling head assembly (20) and to a body (72) of actuator handle assembly (70), thereby providing a mechanical ground for the actuating components therein. The proximal end of driver actuator (64) is coupled to a trigger actuation assembly (84) of actuator handle assembly (70), described below. The distal end of driver actuator (64) is coupled to staple driver (24) such that the rotation of trigger (74) longitudinally actuates staple driver (24). As shown in FIGS. 2A-2C, driver actuator (64) comprises a tubular member having an open longitudinal axis such that actuator (39) coupled to trocar (38) may actuate longitudinally within and relative to driver actuator (64). Of course it should be understood that other components may be disposed within driver actuator (64) as will be apparent to one of ordinary skill in the art in view of the teachings herein.

Shaft assembly (60) may be further constructed in accordance with at least some of the teachings of U.S. Pat. No. 5,205,459; U.S. Pat. No. 5,271,544; U.S. Pat. No. 5,275,322; U.S. Pat. No. 5,285,945; U.S. Pat. No. 5,292,053; U.S. Pat. No. 5,333,773; U.S. Pat. No. 5,350,104; U.S. Pat. No. 5,533,661; and/or in accordance with other configurations as will be apparent to one of ordinary skill in the art in view of the teachings herein.

### D. Exemplary Actuator Handle Assembly

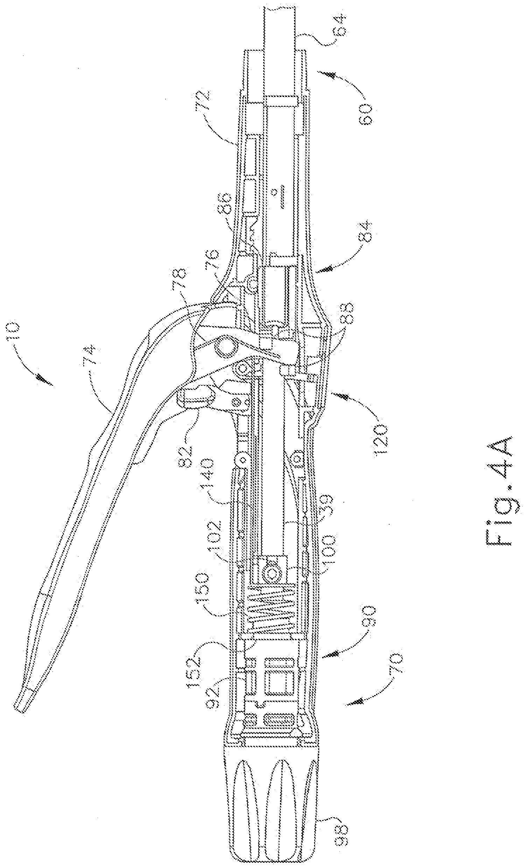

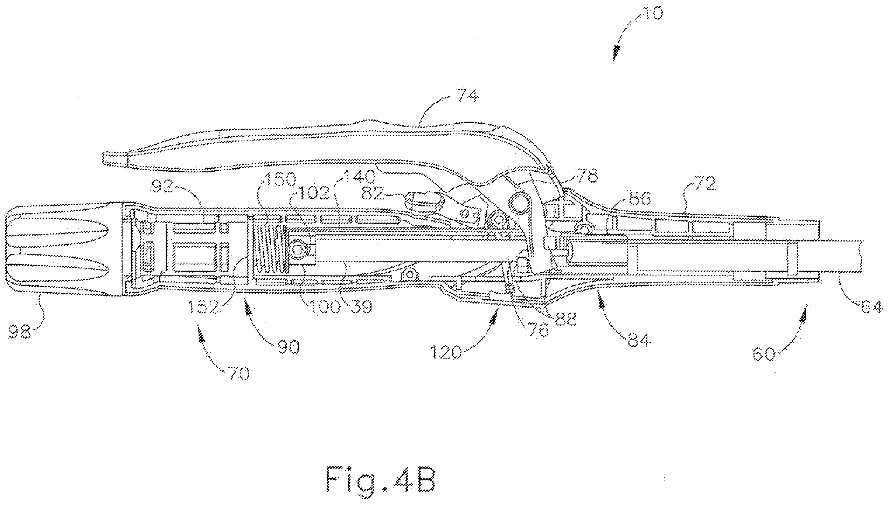

Referring now to FIGS. 4A-5, actuator handle assembly (70) comprises a body (72), a trigger (74), a lockout feature (82), a trigger actuation assembly (84), and a trocar actuation assembly (90). Trigger (74) of the present example is pivotably mounted to body (72) and is coupled to trigger actuation assembly (84) such that rotation of trigger (74) from an unfired position (shown in FIG. 4A) to a fired position (shown in FIG. 4B) actuates driver actuator (64) described above. A spring (78) is coupled to body (72) and trigger (74) to bias trigger (74) towards the unfired position. Lockout feature (82) is a pivotable member that is coupled to body (72). In a first, locked position, lockout feature (82) is pivoted upwards and away from body (72) such that lockout feature (82) engages trigger (74) and mechanically resists actuation of trigger (74) by a user. In a second, unlocked position, such as that shown in FIGS. 1 and 4B, lockout feature (82) is pivoted downward such that trigger (74) may be actuated by the user. Accordingly, with lockout feature (82) in the second position, trigger (74) can engage a trigger actuation assembly (84) to fire instrument (10).

As shown in FIGS. 4A-4B, trigger actuation assembly (84) of the present example comprises a slidable trigger carriage (86) engaged with a proximal end of driver actuator (64). Carriage (86) includes a set of tabs (88) on a proximal end of carriage (86) to retain and engage a pair of trigger arms (76) extending from trigger (74). Accordingly, when trigger (74) is pivoted, carriage (86) is actuated longitudinally and transfers the longitudinal motion to driver actuator (64). In the example shown, carriage (86) is fixedly coupled to the proximal end of driver actuator (64), though this is merely optional. Indeed, in one merely exemplary alternative, carriage (86) may simply abut driver actuator (64) while a distal spring (not shown) biases driver actuator (64) proximally relative to actuator handle assembly (70).

Trigger actuation assembly (84) may be further constructed in accordance with at least some of the teachings of U.S. Pat. No. 5,205,459; U.S. Pat. No. 5,271,544; U.S. Pat. No. 5,275,322; U.S. Pat. No. 5,285,945; U.S. Pat. No. 5,292,053; U.S. Pat. No. 5,333,773; U.S. Pat. No. 5,350,104; U.S. Pat. No. 5,533,661; and/or in accordance with other configurations as will be apparent to one of ordinary skill in the art in view of the teachings herein.

Body (72) also houses a trocar actuation assembly (90) configured to actuate trocar (38) longitudinally in response to rotation of adjusting knob (98). As best shown in FIGS. 4A-5, trocar actuation assembly (90) of the present example comprises adjusting knob (98), a grooved shank (94), and a sleeve (92). Grooved shank (94) of the present example is located at a distal end of trocar actuator (39), though it should be understood that grooved shank (94) and trocar actuator (39) may alternatively be separate components that engage to transmit longitudinal movement. Adjusting knob (98) is rotatably supported by the proximal end of body (72) and is operable to rotate sleeve (92) that is engaged with grooved shank (94) via an internal tab (not shown). Grooved shank (94) of the present example comprises a continuous groove (96) formed in the outer surface of grooved shank (94). Accordingly, when adjusting knob (98) is rotated, the internal tab rides within groove (96) and grooved shank (94) is longitudinally actuated relative to sleeve (92). Since grooved shank (94) is located at the distal end of trocar actuator (39), rotating adjusting knob (98) in a first direction advances trocar actuator (39) distally relative to actuator handle assembly (70). Accordingly, the gap distance *d* between anvil (40) and stapling head assembly (20) is increased. By rotating adjusting knob (98) in the opposite direction, trocar actuator (39) is actuated proximally relative to actuator handle assembly (70) to reduce the gap distance *d* between anvil (40) and stapling head assembly (20). Thus, trocar actuation assembly (90) is operable to actuate trocar (38) in response to rotating adjustment knob (98). Of course other configurations for trocar actuation assembly (90) will be apparent to one of ordinary skill in the art in view of the teachings herein.

Groove (96) of the present example comprises a plurality of different portions (96A, 96B, 96C) that have a varying pitch or number of grooves per axial distance. The present groove (96) is divided into a distal portion (96A), a middle portion (96B) and a proximal portion (96C). As shown in FIG. 5, distal portion (96A) comprises a fine pitch or a high number of grooves over a short axial distance of grooved shank (94) such that a large number of rotations of adjusting knob (98) are required to traverse the short axial distance. Middle portion (96B) comprises a section with comparably coarser pitch or fewer grooves per axial distance such that relatively few rotations are required to traverse a long axial distance. Accordingly, the gap distance *d* may be quickly reduced through relatively few rotations of adjusting knob (98). Proximal portion (96C) of the present example is substantially similar to distal portion (96A) and comprises a fine pitch or a high number of grooves over a short axial distance of grooved shank (94) such that a large number of rotations are required to traverse the short axial distance. Proximal portion (96C) of the present example is positioned within sleeve (92) when anvil (40) is substantially near to stapling head assembly (20) such that indicator bar (110) moves within indicator window (120) along scale (130) to indicate that the anvil gap is within a desired operating range, as will be described in more detail below. Accordingly, when the tab is within proximal portion (96C) of groove (96), each rotation of adjusting knob (98) may reduce the gap distance *d* by a small amount to provide for fine tuning.

Trocar actuation assembly (90) may be further constructed in accordance with at least some of the teachings of U.S. Pat. No. 5,205,459; U.S. Pat. No. 5,271,544; U.S. Pat. No. 5,275,322; U.S. Pat. No. 5,285,945; U.S. Pat. No. 5,292,053; U.S. Pat. No. 5,333,773; U.S. Pat. No. 5,350,104; U.S. Pat. No. 5,533,661; and/or in accordance with other configurations as will be apparent to one of ordinary skill in the art in view of the teachings herein.

In the example shown in FIGS. 4A-4B, a U-shaped clip (100) is attached to an intermediate portion of trocar actuator (39) located distally of grooved shank (94). U-shaped clip (100) engages with a portion of body (72) to substantially prevent trocar actuator (39) from rotating about its axis when adjusting knob (98) is rotated. U-shaped clip (100) further includes an elongated slot (102) on each of its opposite sides for receiving an attachment member, such as a screw, bolt, pin, clip, etc., to selectively adjust the longitudinal position of elongated slot (102) of U-shaped clip (100) relative to trocar actuator (39) for purposes of calibrating indicator bar (110) relative to scale (130).

As shown in FIG. 5, actuator handle assembly (70) further includes an indicator bracket (140) configured to engage and pivot an indicator (104). Indicator bracket (140) of the present example is slidable relative to body (72) along a pair of slots formed on body (72). Indicator bracket (140) comprises a rectangular plate (144), an indicator arm (146), and an angled flange (142). Angled flange (142) is formed at the proximal end of rectangular plate (144) and includes an aperture (not shown) to slidable mount onto trocar actuator (39) and/or grooved shank (94). A coil spring (150) is interposed between flange (142) and a boss (152) to bias flange (142) against U-shaped clip (100). Accordingly, when U-shaped clip (100) actuates distally with trocar actuator (39) and/or grooved shank (94), coil spring (150) urges indicator bracket (140) to travel distally with U-shaped clip (100). In addition, U-shaped clip (100) urges indicator bracket (140) proximally relative to boss (152) when trocar actuator (39) and/or grooved shank (94) translate proximally, thereby compressing coil spring (150). Of course, it should be understood that in some versions indicator bracket (140) may be fixedly attached to trocar actuator (39) and/or grooved shank (94).

In the present example, a portion of lockout feature (82) abuts a surface (141) of indicator bracket (140) when indicator bracket (140) is in a longitudinal position that does not correspond to when the anvil gap is within a desired operating range (e.g., a green colored region or "green zone"). When the anvil gap is within a desired operating range (e.g., a green colored region or "green zone"), indicator bracket (140) narrows to provide a pair of gaps (145) on either side of an indicator arm (146) that permits lockout feature (82) to pivot, thereby releasing trigger (74). Accordingly, lockout feature (82) and indicator bracket (140) can substantially prevent a user from releasing and operating trigger (74) until anvil (40) is in a predetermined operating range. Of course it should be understood that lockout feature (82) may be omitted entirely in some versions.

This operating range may be visually communicated to the user via an indicator bar (110) of an indicator (104) shown against a scale (130), described briefly above. At the distal end of indicator bracket (140) is a distally projecting indicator arm (146) which terminates at a laterally projecting finger (148) for controlling the movement of indicator (104). Indicator arm (146) and finger (148), best shown in FIG. 5, are configured to engage a tab (106) of indicator (104) such that indicator (104) is pivoted when indicator bracket (140) is actuated longitudinally. In the present example, indicator (104) is pivotably coupled to body (72) at a first end of indicator (104), though this is merely optional and other pivot points for indicator (104) will be apparent to one of ordinary skill in the art in view of the teachings herein. An indicator bar (110) is positioned on the second end of indicator (104) such that indicator bar (110) moves in response to the actuation of indicator bracket (140). Accordingly, as discussed above, indicator bar (110) is displayed through an indicator window (120) against a scale (130) (shown in FIG. 6) to show the relative gap distance *d* between anvil (40) and stapling head assembly (20).

Of course indicator bracket (140), indicator (104), and/or actuator handle assembly (70) may be further constructed in accordance with at least some of the teachings of U.S. Pat. No. 5,205,459; U.S. Pat. No. 5,271,544; U.S. Pat. No. 5,275,322; U.S. Pat. No. 5,285,945; U.S. Pat. No. 5,292,053; U.S. Pat. No. 5,333,773; U.S. Pat. No. 5,350,104; U.S. Pat. No. 5,533,661; and/or in accordance with other configurations as will be apparent to one of ordinary skill in the art in view of the teachings herein.

### II. Exemplary Staple Cartridge

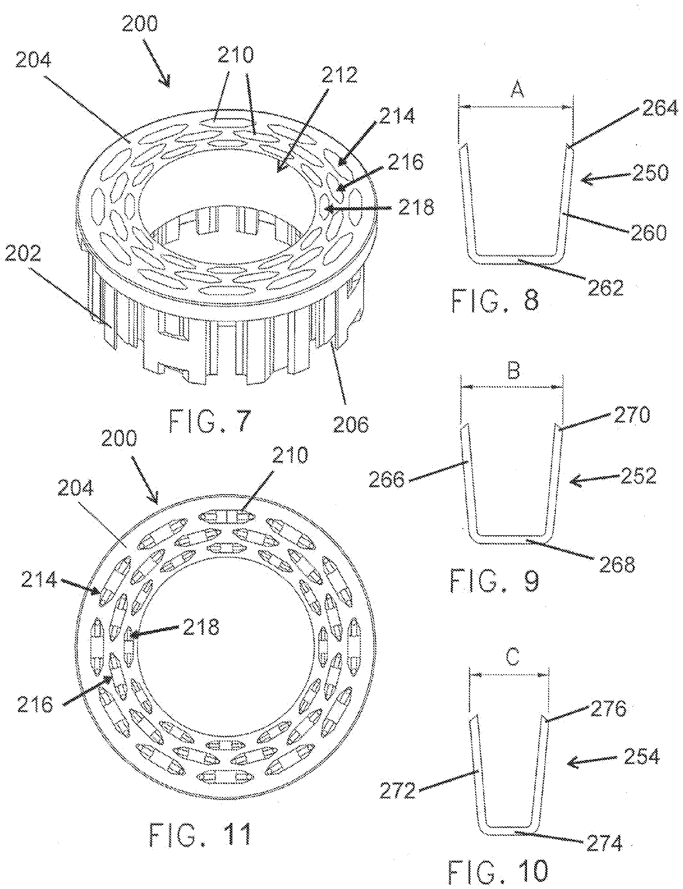

During operation of instrument (10), a staple cartridge (200) may be used to hold staples (66) in preparation for firing in a plurality of concentrically arranged rings. In some instances, each ring of staples (66) within staple cartridge (200) may have an equal number of staples (66), even when the rings vary in radius, by varying the total width of each staple within a ring of staples. It will be appreciated that varying the widths of staples (66) may allow an inner ring of concentric rings of staples (66) to be more tightly packed than an outer ring. It will also be appreciated that providing an equal number of staples (66) with each concentric ring may allow for better hemostasis once staples (66) are driven into tissue. Other desirable traits may result as well. FIG. 7 shows an exemplary staple cartridge (200) operable for use with instrument (10), in which three rings of staples include the same number of staples in each ring. Generally speaking, staple cartridge (200) is operable to hold a plurality of staples (250, 252, 254) (shown in FIGS. 8-10) having different shapes and sizes as will be described in further detail below. In some instances, staples (250, 252, 254) may also be positioned within staple cartridge (200) with other supplements and/or components that may be configured to be fired with staples (250), which will be discussed further below. Of course, it will be understood that staples (250, 252, 254) may be loaded into staple cartridge (200) alone without additional components.

Staple cartridge (200) comprises an upper ring (204) and lower body (202). In the exemplary version, staple cartridge (200) has an elongated ring shape, but it will be understood that staple cartridge (200) may have any suitable shape as would be apparent to one of ordinary skill in the art in view of the teachings herein. Upper ring (204) defines a plurality of staple apertures (210) that encircle upper ring (204). Staple apertures (210) have an elongated hexagonal shape having rounded ends as seen in FIG. 7 and further in FIG. 11. It will be understood that staple apertures (210) may have other shapes such as an elongated rectangle, elliptical, circular, square, slit shaped, or any other suitable shape for receiving one or more staples as would be apparent to one of ordinary skill in the art in view of the teachings herein.

As also seen in FIGS. 7 and 11, three rings of staple apertures (210) encircle upper ring (204). An outer ring (214), a middle ring (216), and an inner ring (218) make up the annular rows of staple apertures (210). Outer ring (214) of staple apertures (210) has the largest staple apertures (210), and as a result, it will be understood that outer ring (214) has the lowest density of or fewest staple apertures (210). Inner ring (218) has the smallest sized apertures (210) such that inner ring (218) has the highest density of staple apertures (210). Middle ring (216) is positioned between outer ring (214) and inner ring (218). Middle ring (216) includes staple apertures (210) sized between staple apertures (210) of outer ring (214) and inner ring (218). As a result, middle ring (216) has a staple aperture (210) density between outer ring (214) and inner ring (218). Outer ring (214), middle ring (216) and inner ring (218) are positioned concentrically about an opening (212) defined by staple cartridge (200). In some versions, the number of staple apertures (210) of inner ring (218) is equal to the number of staple apertures (210) of middle ring (216), and is equal to the number of staple apertures (210) of outer ring (214). However, it will be understood that the number of staple apertures (210) may also be varied between rings (214, 216, 218) in some versions. Furthermore, outer ring (214), middle ring (216), and inner ring (218) are positioned such that staple apertures (210) of middle ring (216) are weaved between staple apertures (210) of outer ring (214) and inner ring (218). It will be understood that other suitable configurations of positioning outer ring (214), middle ring (216), and inner ring (218) may be used as would be apparent to one of ordinary skill in the art. For instance, while middle ring (216) roughly splits the distance between inner ring (218) and outer ring (214), it will be understood that middle ring (216) may be positioned more closely to inner ring (218) or more closely to outer ring (214). It will further be understood that while outer ring (214), middle ring (216), and inner ring (218) comprise staple apertures (210) arranged in a ring-like fashion about upper ring (204) in the exemplary version, other arrangements for staple apertures (210) may be used. For instance, staple apertures (210) may be arranged in an elliptical or square-shaped manner about upper ring (204). Indeed, staple apertures (210) may be arranged in any suitable fashion around upper ring (204) as would be apparent to one of ordinary skill in the art in view of the teachings herein.

Staple cartridge (200) fits into tubular casing (22) of stapling head assembly (20). In particular, lower body (202) provides a friction fit with tubular casing (22). It will be understood that other suitable ways of connecting staple cartridge (200) and stapling head assembly (20) may be used as would be apparent to one of ordinary skill in the art in view of the teachings herein. For example, staple cartridge (200) may be clipped to, attached via snap fitting, screwed in, or otherwise selectively connected to stapling head assembly (20) in any other suitable manner. Lower body (202) of staple cartridge (200) also defines channels (206) operable to receive complementary features of staple driver (24). Channels (206) are in communication with staple apertures (210) such that the complementary features of staple driver (24) can advance through channels (206) to drive staples (250, 252, 254). Opening (212) is sized to receive cylindrical knife (36), which is driven through opening (212) to sever tissue in a manner as described above when tissue is clamped between stapling head assembly (20) and anvil (40).

FIG. 8 shows large staple (250), which may be positioned within staple apertures (210) of outer ring (214). Large staple (250) comprises a crown (262) and legs (260) extending from crown (262). As seen in the illustrated version, legs (260) flare outward such that legs (260) define a distance, "A," between legs (260), where "A" is wider than crown (262). Each leg (260) terminates in an angled tip (264) operable to aid leg (260) of large staple (250) in being pushed through tissue. It will be understood that the width of legs (260) at distance "A" corresponds to the size of staple apertures (210) in outer ring (214). In some instances, "A" is slightly larger than the width of staple apertures (210) of outer ring (214), such that large staple (250) is releasably secured in staple apertures (210) by a slight interference fit.

FIG. 9 shows medium staple (252), which may be positioned within staple apertures (210) of middle ring (216). Medium staple (252) comprises a crown (268) and legs (266) extending from crown (268). As seen in the illustrated version, legs (266) flare outward such that legs (266) define a distance, "B," between legs (266), where "B" is wider than crown (268). Legs (266) terminate in an angled tip (270) operable to aid medium staple (252) in being pushed through tissue. It will be understood that the width of legs (266) at distance "B" corresponds to the size of staple apertures (210) in middle ring (216). In some instances, "B" is slightly larger than the width of staple apertures (210) of middle ring (216), such that medium staple (252) is releasably secured in staple apertures (210) by a slight interference fit.

FIG. 10 shows small staple (254), which may be positioned within staple apertures (210) of inner ring (218). Small staple (254) comprises a crown (274) and legs (272) extending from crown (274). As seen in the illustrated version, legs (272) flare outward such that legs (272) define a distance, "C," between legs (272), where "C" is wider than crown (274). Legs (272) terminate in an angled tip (276) operable to aid small staple (254) in being pushed through tissue. It will be understood that the width of legs (272) at distance "C" corresponds to the size of staple apertures (210) in inner ring (218). In some instances, "C" is slightly larger than the width of staple apertures (210) of inner ring (218), such that small staple (254) is releasably secured in staple apertures (210) by a slight interference fit.

As seen in FIGS. 8-10, large staple (250) has crown (262) that is longer than crown (268) of medium staple (252) and crown (274) of small staple (254), where crown of medium staple (252) is longer than crown (274) of small staple (254). It will be understood that relative widths of staples (250, 252, 254) are such that small staples (254) may be arranged within staple apertures (210) such that small staples (254) have a relatively high staple density about staple cartridge (200). Accordingly, medium staples (252) and large staples (250) may be arranged about staple cartridge such that medium staples (252) are less densely spaced and large staples (250) are least densely spaced. As a result of the above described progressive reduction in crown (262, 268, 274) lengths, the number of staples (250, 252, 254) in each ring (214, 216, 218) may be equal in number.

While legs (260, 266, 272) are shown to be flared out, it will be understood that legs (260, 266, 272) may be perpendicular to crown (262, 268, 274), angled acutely in relation to crown (262, 268, 274), or oriented at any other suitable angle in relation to crown (262, 268, 274) as would be apparent to one of ordinary skill in the art in view of the teachings herein. Furthermore, it will be understood that legs (260, 266, 272) need not necessarily have a straight shape. For instance, legs (260, 266, 272) may be curved, bent, or any other suitable shape operable to aid staples (250, 252, 254) to be driven through tissue. Additionally, while FIGS. 8-10 show staples (250, 252, 254) having legs (260, 266, 272) of generally equal thickness, it will be understood that legs (260, 266, 272) may be of different thicknesses. For instance, large staple (250) may have legs (260) thicker or thinner than legs (272, 266) of small staple (254) or medium staple (252). In yet other versions, rather than having a uniform thickness, legs (260, 266, 272) may have varying thicknesses. For instance, legs (260, 266, 272) may be thicker or thinner near crown (262, 268, 274) or near tip (264, 270, 276) or any other suitable variation as would be apparent to one of ordinary skill in the art in view of the teachings herein. While the exemplary version shows staple cartridge (200) having three rings (214, 216, 218) of staples (250, 252, 254), it will be understood that two rings, or more than three rings of staples may be used.

FIG. 11 shows staples (250, 252, 254) placed within staple apertures (210) of staple cartridge (200). It will be understood that staples (250, 252, 254) may come preloaded, but in other versions, it will be understood that staples (250, 252, 254) may be loaded into staple cartridge (200) at any suitable time prior to using stapling instrument (10).

In one exemplary use of staple cartridge (200), staple cartridge (200) may be loaded as seen in FIG. 11. Lower body (202) may be inserted into tubular casing (22). Tubular member (44) is positioned through opening (212) of staple cartridge (200). Furthermore, circular knife (36) is operable to translate through opening (212) of staple cartridge (200). Staple driver (24) is positioned relative to staple cartridge (200) such that as staple driver (24) translates distally, staple driver (24) advances through channel (206) to drive staples (250, 252, 254) distally. As a result, when trigger (74) is rotated, staple driver (24) actuates longitudinally, which drives staples (250, 252, 254) distally into tissue. Legs (260, 266, 272) of staples (250, 252, 254) contact staple forming pockets (52) of anvil head (48), thereby bending legs (260, 266, 272) toward their respective crowns (262, 268, 274). Circular knife (36) also translates to cut tissue. Circular knife (36) then retracts and staple driver (24) retracts. Staples (250, 252, 254) remain in tissue where they secure an anastomosis between two bodily lumens in a patient (e.g., securing two ends of an esophagus, colon, or other GI tract portion together, etc.). It will be understood that once driven into tissue, the ring of staples formed by the outermost large staples (250) may function to structurally secure the anastomosis whereas the ring of staples formed by medium staples (252) or small staples (254) function to prevent leaking of the anastomosis. In other words, large staple (250) may primarily serve a role of structural reinforcement while medium staple (252) and/or small staple (254) may primarily serve a role of providing a fluid-tight seal. Staples (250, 252, 254) may be operable to dissolve at a later point in time.

### III. Exemplary Staple Support Element

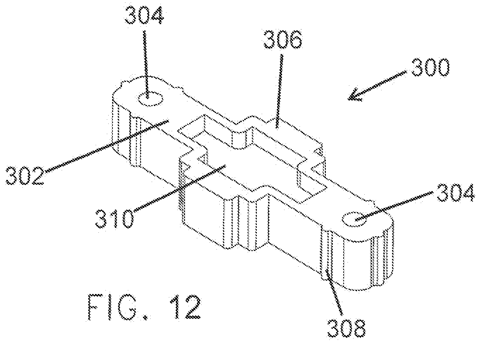

It will be understood that as staples (250, 252, 254) are driven into tissue, in some instances, given the thickness of staples (250, 252, 254), staples (250, 252, 254) may have a tendency fall through or otherwise fail to remain anchored in tissue. FIG. 12 depicts an exemplary staple pledget or support element (300) that may be used to effectively increase the footprint size of a staple crown, thereby reducing a risk that the crown might otherwise pull through tissue. Support element (300) of this example comprises a support element body (302) that defines a pair of openings (304) operable to receive legs (260, 266, 272) of staples (250, 252, 254). Body (302) further defines a body opening (310) extending through the body of staple support element (300). It will be understood that staple support element (300) may be used to prevent staples (250, 252, 254) from tearing through tissue. Furthermore, staple support element (300) may be operable to reduce leak paths in the area where staples (250, 252, 254) are inserted into tissue and may further be operable to absorb leaking in the event that leaking does occur. Staple support element (300) is dimensioned such that staple support element (300) may be placed on staples (250, 252, 254) such that staples (250, 252, 254) may still be placed within staple apertures (210) shown, for instance, in FIG. 11. Furthermore, it will be understood that staple support element (300) may be sized differently to fit the different sizes of staples (250, 252, 254). Staple support element (300) may be constructed of any suitable material operable to prevent leaking or to absorb leaking fluid from tissue around the application of staple (250, 252, 254). For instance, staple support element (300) may be constructed of a polymer material or in addition to or in the alternative may be constructed of an absorptive material. Other suitable materials for staple support element (300) may be included as would be apparent to one of ordinary skill in the art in view of the teachings herein.

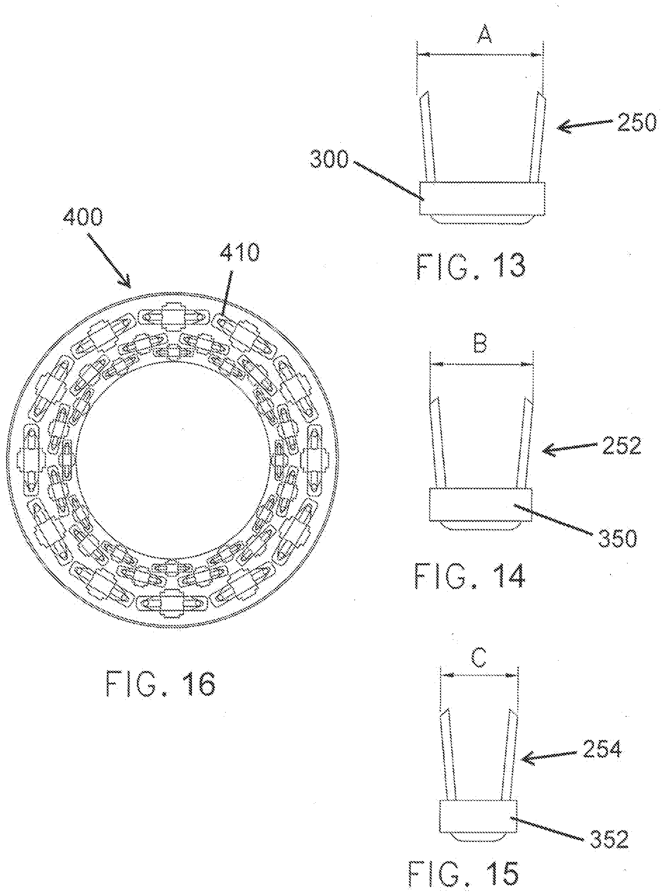

Openings (304) are positioned to line up with legs (260, 266, 272) of staples (250, 252, 254) as shown when fitted in FIGS. 13-15. Openings (304) have a circular shape, but it will be understood that openings (304) may have any suitable shape. For instance, openings (304) may have a square, elliptical, or any other suitable shape for receiving legs (260, 266, 272). Once legs (260, 266, 272) are inserted into openings (304), openings (304) are shaped to be tight enough or spaced such that a friction fit retains body (302) upon staples (250, 252, 254). Body opening (310) is positioned to line up approximately with crowns (262, 268, 274) of staples (250, 252, 254). In the exemplary version, body opening (310) has a widened cross shape. In other versions, it will be understood that body opening (310) may have any suitable shape. For instance, body opening (310) may have a circular, square or any suitable shape. In some versions, it will be understood that body opening (310) may be omitted.

Staple support element (300) further includes wings (306) extending outwardly from body (302). While wings (306) in the exemplary version have a block-like shape, other suitable shapes may be used. By way of example only, wings (306) may have a rounded shape or triangular shape. The exemplary version shows wings (306) on opposing sides of body opening (310), but it will be understood that wings (306) may be positioned at any suitable area around staple support element (300). Furthermore, wings (306) may be constructed such that more than two wings (306) may be positioned around staple support element (300). Other suitable variations will be apparent to one of ordinary skill in the art in view of the teachings herein.

Staple support element (300) further comprises a plurality of ridges (308) positioned near openings (304). Ridges (308) are positioned equidistantly around openings (304), but it will be understood that ridges (308) may be positioned in any suitable manner about openings (304). In some versions, ridges (308) may be excluded entirely.

FIG. 13 shows staple support element (300) outfitted on large staple (250). FIG. 14 shows a medium staple support element (350), which is substantially identical to staple support element (300), but scaled down to fit medium staple (252). Medium staple support element (350) may be outfitted on medium staple (252) as seen in FIG. 14. FIG. 15 shows a small staple support element (352), which is substantially identical to staple support element (300), but scaled down to fit small staple (254). Small staple support element (352) may be outfitted on small staple (254) as seen in FIG. 15. It will be understood that staple support elements (300, 350, 352) may have yet other sizes not shown in the illustrated version to accommodate larger or smaller staples. Furthermore, in some versions, only one set of staples (e.g. large staples (250)) is fitted with support elements (300) while the other staples (e.g. staples (252, 254)) are not. Other suitable variations of outfitting only some staples with support elements (300) will be apparent to one of ordinary skill in the art in view of the teachings herein.

FIG. 16 shows an exemplary staple cartridge (400) with staples (250, 252, 254) loaded into staple apertures (410) of staple cartridge (400). It will be understood that staple apertures (410) may be the same size as staple apertures (210) shown in FIG. 7, but it will also be appreciated that staple apertures (410) may be shaped to complement staples (250, 252, 254) outfitted with staple support elements (300). Furthermore, staple cartridge (400) may be otherwise configured and operable in a manner substantially identical to that described above for staple cartridge (200). It will be understood that staple support elements (300) with staples (250, 252, 254) may be operable to provide greater structural integrity of an anastomosis and may further be operable to reduce risks of leakage by minimizing the number and size of potential leak paths at the anastomosis.

### IV. Exemplary Staple Support Element with Tabs

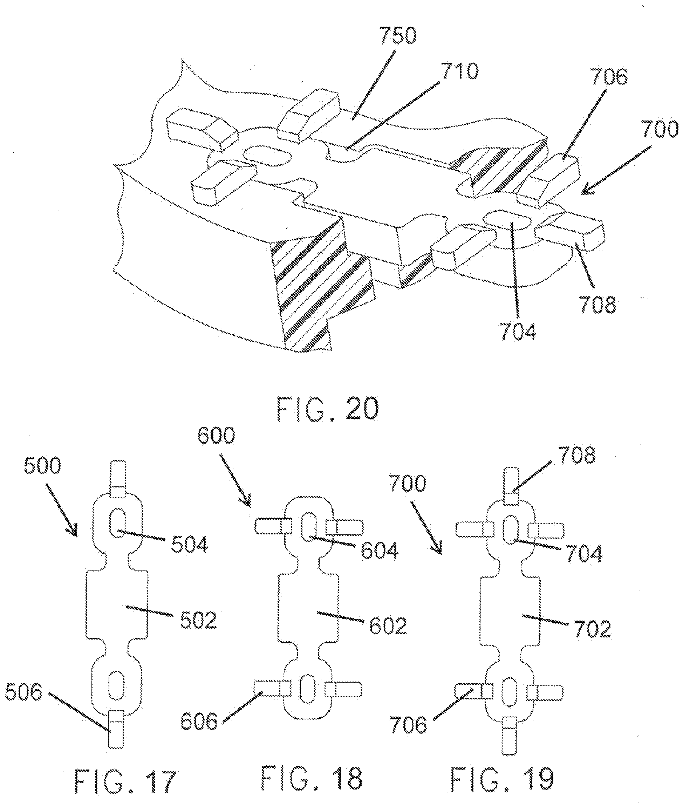

FIG. 17 shows an alternative exemplary version of a staple support element (500) having a body (502), openings (504), and longitudinally oriented tabs (506). Longitudinally oriented tabs (506) extend outwardly from body (502) along a longitudinal axis defined by body (502). Tabs (506) are operable to increase the footprint of staple support element (500) to further reduce a risk that staples (250, 252, 254) might otherwise inadvertently slip through tissue. As can be seen in FIG. 17, due to the inclusion of tabs (506), staple support element (500) has a lengthened footprint. Tabs (506) may be operable to provide greater structural integrity of an anastomosis in conjunction with support element (500) and staples (250, 252, 254) and may further be operable to reduce risks of leakage by minimizing the number and size of potential leak paths at the anastomosis.

FIG. 18 shows an alternative exemplary version of a staple support element (600) having a body (602), openings (604), and transversely oriented tabs (606). It will be understood that transversely oriented tabs (606) may be operable to provide a widened footprint for staple support element (600). While the exemplary version shows staple support element (600) having four transversely oriented tabs (606) it will be understood that any suitable number of tabs (606) may be used. FIG. 19 shows an alternative exemplary version of a staple support element (700) having a body (702), openings (704), and a combination of transversely oriented tabs (706) and longitudinally oriented tabs (708). Due to transversely oriented tabs (706) and longitudinally oriented tabs (708), it will be understood that staple support element (700) has both a wider and longer footprint. As discussed above, tabs (606) with support element (600) may be operable to provide greater structural integrity of an anastomosis in conjunction with staples (250, 252, 254) and may further be operable to reduce risks of leakage by minimizing the number and size of potential leak paths at the anastomosis.

FIG. 20 shows staple support element (700) placed within an aperture (710) of a staple cartridge (750). Horizontal tabs (706) and vertical tabs (708) are operable to fit on the outside of aperture (710) such that tabs (706, 708) do not fall through aperture (710). Staple support element (700) may be loaded with a staple (not shown) passing through openings (704). Upon firing the staple loaded with staple support element (700), both the staple and support element (700) leave aperture (710) of staple cartridge (750) to engage tissue.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI™ system by Intuitive Surgical, Inc., of Sunnyvale, California. Similarly, those of ordinary skill in the art will recognize that various teachings herein may be readily combined with various teachings of U.S. Pat. No. 6,783,524, entitled "Robotic Surgical Tool with Ultrasound Cauterizing and Cutting Instrument," published August 31,2004.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus comprising:
(a) a staple cartridge (750) having a plurality of staple apertures (710);
(b) a plurality of staples, each staple configured to fit within a respective aperture (710) of the staple cartridge (750), the plurality of staples arranged in an annular arrangement about the staple cartridge (750), wherein the plurality of staples are configured to be driven into tissue, wherein each of the plurality of staples comprises a crown and legs extending from the crown in a first direction, the crown having a length defining a longitudinal direction and a width defining a transverse direction;
(c) at least one pledget (700), each pledget (700) configured to engage the legs of a respective staple of the plurality of staples, wherein the at least one pledget (700) is configured to slide along the legs of the respective staple toward the crown, wherein the at least one pledget (700) has a width larger than the crown; and
(d) at least one tab (706, 708) in communication with the at least one pledget (700), wherein the at least one tab (706, 708) extends outwardly from the at least one pledget (700), wherein the at least one tab (706, 708) is oriented longitudinally or transversely in relation to the at least one pledget (700), **characterised in that**:
the at least one pledget (700) is configured to fit within a respective staple aperture (710), and
the at least one tab (706, 708) is configured to be positioned outside of the staple aperture (710) such that the at least one tab (706, 708) does not fall through the staple aperture (710).

2. The apparatus of claim 1, wherein the at least one pledget (700) comprises at least a first pledget and a second pledget, wherein the first pledget has a larger cross section than the second pledget.

3. The apparatus of claim 1, wherein the at least one pledget (700) defines a pair of openings (704) operable to receive legs of the respective staple

4. The apparatus of claim 1, wherein the at least one tab (706, 708) includes a combination of transversely oriented and longitudinally oriented tabs (706, 708).

## Patentansprüche

1. Vorrichtung, umfassend:
(a) ein Klammermagazin (750) mit einer Vielzahl von Klammeröffnungen (710),
(b) eine Vielzahl von Klammern, wobei jede Klammer dazu ausgestaltet ist, in eine jeweilige Öffnung (710) des Klammermagazins (750) zu passen, wobei die Vielzahl von Klammern in einer kreisförmigen Anordnung um das Klammermagazin (750) angeordnet sind, wobei die Vielzahl von Klammern zum Eintreiben in Gewebe ausgestaltet sind, wobei jede der Vielzahl von Klammern einen Rücken und sich vom Rücken in einer ersten Richtung erstreckende Beine umfasst, wobei der Rücken eine Länge hat, die eine Längsrichtung definiert, sowie eine Breite, die eine Querrrichtung definiert,
(c) mindestens ein Pledget (700), wobei jedes Pledget (700) dazu ausgestaltet ist, die Beine einer jeweiligen Klammer der Vielzahl von Klammern in Eingriff zu nehmen, wobei das mindestens eine Pledget (700) dazu ausgestaltet ist, entlang den Beinen der jeweiligen Klammer zum Rücken hin zu gleiten, wobei das mindestens eine Pledget (700) eine Breite hat, die größer als der Rücken ist, und
(d) mindestens eine Lasche (706, 708) in Verbindung mit dem mindestens einen Pledget (700), wobei sich die mindestens eine Lasche (706, 708) von dem mindestens einen Pledget (700) nach außen erstreckt, wobei die mindestens eine Lasche (706, 708) in Bezug auf das mindestens eine Pledget (700) längs oder quer ausgerichtet ist, **dadurch gekennzeichnet, dass**
das mindestens eine Pledget (700) dazu ausgestaltet ist, in eine jeweilige Klammeröffnung (710) zu passen, und
die mindestens eine Lasche (706, 708) dazu ausgestaltet ist, außerhalb der Klammeröffnung (710) positioniert zu werden, so dass die mindestens eine Lasche (706, 708) nicht durch die Klammeröffnung (710) fällt.

2. Vorrichtung nach Anspruch 1, wobei das mindestens eine Pledget (700) mindestens ein erstes Pledget und ein zweites Pledget umfasst, wobei das erste Pledget einen größeren Querschnitt als das zweite Pledget hat.

3. Vorrichtung nach Anspruch 1, wobei das mindestens eine Pledget (700) ein Paar zur Aufnahme von Beinen der jeweiligen Klammer betreibbare Öffnungen (704) definiert.

4. Vorrichtung nach Anspruch 1, wobei die mindestens eine Lasche (706, 708) eine Kombination aus quer ausgerichteten und längs ausgerichteten Laschen (706, 708) aufweist.

## Revendications

1. Appareil comprenant :
(a) une cartouche (750) d'agrafes comportant une pluralité d'ouvertures (710) pour agrafe ;
(b) une pluralité d'agrafes, chaque agrafe étant configurée pour entrer dans une ouverture (710) respective de la cartouche (750) d'agrafes, la pluralité d'agrafes étant agencées selon un agencement annulaire autour de la cartouche (750) d'agrafes,
dans lequel la pluralité d'agrafes sont configurées pour être enfoncées dans un tissu,
dans lequel chaque agrafe de la pluralité d'agrafes comprend une couronne et des jambes s'étendant depuis la couronne dans une première direction, la couronne ayant une longueur définissant une direction longitudinale et une largeur définissant une direction transversale ;
(c) au moins une petite compresse (700), chaque petite compresse (700) étant configurée pour entrer en prise avec les jambes d'une agrafe respective de la pluralité d'agrafes,
dans lequel ladite petite compresse (700) est configurée pour coulisser vers la couronne le long des jambes de l'agrafe respective,
dans lequel ladite petite compresse (700) a une largeur supérieure à la couronne ; et
(d) au moins une languette (706, 708) en liaison avec ladite petite compresse (700),
dans lequel ladite languette (706, 708) s'étend vers l'extérieur depuis ladite petite compresse (700),
dans lequel ladite languette (706, 708) est orientée longitudinalement ou transversalement par rapport à ladite petite compresse (700),
**caractérisé en ce que** :
ladite petite compresse (700) est configurée pour entrer dans une ouverture (710) respective d'agrafes et
ladite languette (706, 708) est configurée pour être mise en place à l'extérieur de l'ouverture (710) d'agrafes de telle sorte que ladite languette (706, 708) ne tombe pas par l'ouverture (710) d'agrafes.

2. Appareil selon la revendication 1, dans lequel ladite petite compresse (700) comprend au moins une première petite compresse et une seconde petite compresse,
dans lequel la première petite compresse a une section transversale supérieure à celle de la seconde petite compresse.

3. Appareil selon la revendication 1, dans lequel ladite petite compresse (700) délimite une paire d'ouvertures (704) servant à recevoir les jambes de l'agrafe respective.

4. Appareil selon la revendication 1, dans lequel ladite languette (706, 708) comprend une combinaison de languettes (706, 708) orientées transversalement et orientées longitudinalement.
